# EUROPÄISCHE PATENTSCHRIFT

(11) **EP 2 700 929 B1**
(45) Veröffentlichungstag und Bekanntmachung des Hinweises auf die Patenterteilung: **12.06.2019**
(21) Anmeldenummer: 13173560.7
(22) Anmeldetag: 25.06.2013
(51) Int. Cl.: G01N 1/12, G01N 33/20

(54) **Messsonde zur Probennahme in Metallschmelzen**
Measuring probe for sampling in metal melts
Sonde de mesure pour le prélèvement d'échantillons dans du métal en fusion

(30) Priorität: 24.08.2012 DE 102012016697
(43) Veröffentlichungstag der Anmeldung: 26.02.2014
(73) Patentinhaber: Heraeus Electro-Nite International N.V., 3530 Houthalen (BE)
(72) Erfinder: Beyens, Dries, 3640 Kinrooi (BE); Bortels, Eric B., 3560 Lummen (BE)
(74) Vertreter: Heraeus IP

(56) Entgegenhaltungen:
- DE-A1- 3 402 818
- SU-A1- 1 481 619
- US-A- 4 499 777

## Beschreibung

Die Erfindung betrifft eine Messsonde zur Probennahme in Metallschmelzen, insbesondere in Stahlschmelzen, mit einem an einem Eintauchende eines Trägerrohres angeordneten Messkopf, wobei der Messkopf mindestens eine Probenkammer aufweist, wobei die Probenkammer einen Einlaufkanal aufweist, dessen eines Ende in der Probenkammer mündet und dessen zweites Ende eine Einlauföffnung aufweist, die aus der dem Trägerrohr abgewandten Stirnseite des Messkopfes herausragt und mit einer Schutzkappe abgedeckt ist.

Derartige Messsonden sind beispielsweise aus DE 102010024282 A1 bekannt. Diese Sonden sind zur Messung in einem Konverter bestimmt. In einen Konverter wird mit einer Lanze Sauerstoff in die Metallschmelze eingeblasen. Gasblasen innerhalb der Metallschmelze, zum Beispiel einer Stahlschmelze, können die Probennahme deutlich stören, da sie in die Probenkammer eindringen können, so dass die Proben fehlerbehaftet sind und schlecht ausgewertet werden können. Ähnliche Probennehmer sind beispielsweise aus DE 102005060493 B3 oder DE 3402818 A1 bekannt.

Aufgabe der vorliegenden Erfindung ist es, die bekannten Probennehmer zu verbessern und qualitativ hochwertige Probennahmen zu ermöglichen.

Die Aufgabe wird gelöst durch die Merkmale des unabhängigen Anspruchs. Vorzugsweise Ausgestaltungen sind in den Unteransprüchen angegeben.

Dadurch, dass außerhalb des Einlaufkanals in Einlaufrichtung vor der Einlauföffnung ein Schutzschild beabstandet von der mit der Schutzkappe abgedeckten Einlauföffnung angeordnet ist, wobei der Schutzschild die Einlauföffnung abdeckt und wobei der Schutzschild den Einlaufkanal seitlich nicht vollständig umgibt, gelangen die in der Metallschmelze vorhandenen Gasblasen, die u.a. durch das Einblasen von Sauerstoff in einem Konverter entstehen, nicht unmittelbar vor die Schutzkappe der Einlauföffnung und somit nach dem Abschmelzen der Schutzkappe nicht in die Probenkammer hinein, sondern sie werden von dem Schutzschild abgeleitet. Das Einblasen von Gas in einen Konverter kann zum einen erfolgen durch Einblasen von Sauerstoff durch eine Blaslanze und zum anderen durch Einblasen von Inertgas durch Bodendüsen. Es hat sich gezeigt, dass durch die Erfindung deutlich bessere, porenfreie Proben gewonnen werden können.

Die Schutzkappe ist eine übliche Schutzkappe, die schmilzt oder sich auflöst, wenn der Messkopf in die Metallschmelze eingetaucht ist, so dass die Einlauföffnung für die Probenkammer freigegeben wird. Für den Schutzschild ist es wesentlich, dass er beabstandet von der Einlauföffnung mit ihrer Schutzkappe angeordnet ist. Der Schutzschild deckt dabei die Einlauföffnung der Gestalt ab, dass ihr Umriss, in Achsrichtung des Einlaufkanals gesehen, den Umriss der Einlauföffnung des Einlaufkanals umgibt. Die Halterung des Schutzschildes selbst wird zweckmäßigerweise an dem Messkopf erfolgen.

Die Halterung und Form des Schutzschildes muss so gestaltet sein, dass eine seitliche Öffnung zwischen Einlauföffnung und Schutzschild besteht, durch die die Metallschmelze in den Einlaufkanal gelangen kann. Seitlich bedeutet hierbei abweichend von der Richtung der Achse des Einlaufkanals, vorzugsweise etwa radial zu dieser Achse.

Vorzugsweise ist der Schutzschild beständiger gegenüber Stahlschmelze als die Schutzkappe der Einlauföffnung. Diese größere Beständigkeit kann durch ein beständigeres Material oder durch eine größere Dicke des Materials erreicht werden. Der Schutzschild ist vorzugsweise an der Stirnseite des Messkopfes angeordnet, wobei er an der Oberfläche des Messkopfes fixiert oder in dem Messkopf selbst gehalten wird.

Der Schutzschild kann insbesondere als abgewinkeltes oder abgebogenes, streifenförmiges Material ausgebildet sein, da dies eine einfache Herstellung einerseits und eine große Öffnung für die einlaufende Metallschmelze andererseits ermöglicht. Der Schutzschild kann aus Metall, Keramik oder aus Quarz gebildet sein.

Zweckmäßig ist es, dass zwischen Schutzschild und Einlaufkanal eine an einer in Richtung Schutzschild gedachten Verlängerung des Einlaufkanals umlaufende Fläche gebildet ist, die größer ist als die Fläche der Einlauföffnung, so dass die in den Einlaufkanal strömende Metallschmelze diesen ungehindert erreichen kann und kein Abriss erfolgt, der eine Blasenbildung nach sich ziehen könnte. Insbesondere kann es zweckmäßig sein, dass der Abstand zwischen Einlauföffnung und Schutzschild in Richtung des Einlaufkanals mindestens halb so groß ist wie der Durchmesser der Einlauföffnung.

Die Messsonde ist vorteilhaft derart ausgebildet, dass die Stirnseite des Messkopfes mit dem Einlaufkanal und dem Schutzschild von einer Seite Schlackekappe abgedeckt ist. Auf diese Weise ist der Schutzschild zwischen der Schlackekappe und der Schutzkappe des Einlaufkanals angeordnet. Die Schlackekappe schützt die Stirnseite des Messkopfes während des Transports der Messsonde und beim Eintauchen durch die Schlacke, so dass keine Beschädigung des Schutzschildes oder des Einlaufkanals durch die Schlacke erfolgen kann.

Die Schlackekappe selbst löst sich spätestens beim Eindringen des Messkopfes in die Metallschmelze auf und gibt danach die Stirnseite des Messkopfes mit daran befindlichem Einlaufkanal für die Metallschmelze frei, so dass sich die Schutzkappe des Einlaufkanals auflöst und Metallschmelze in die Probenkammer eindringen kann. Vorteilhaft ist es, dass an den Messkopf mindestens ein Sensor zur Messung der Temperatur oder eines anderen Parameters der Metallschmelze angeordnet ist. Dieser Sensor ist zweckmäßigerweise ebenfalls an der Stirnseite des Messkopfes angeordnet und von der Schlackekappe abgedeckt.

Nachfolgend wird ein Ausführungsbeispiel der Erfindung anhand einer Zeichnung näher erläutert. In der Zeichnung zeigt
- Figur 1:: die Anwendung der erfindungsgemäßen Messsonde in einem Konverter
- Figur 2:: die schematische Ansicht des Messkopfes der erfindungsgemäßen Messsonde und
- Figur 3:: eine Schnittdarstellung der Messsonde.

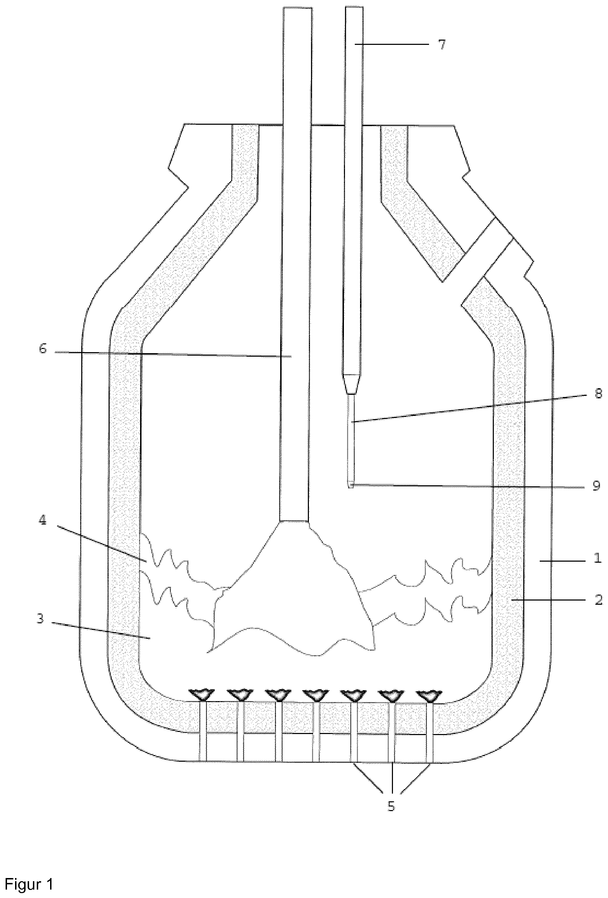

Figur 1 zeigt einen Konverter 1 mit einer Auskleidung 2. In dem Konverter 1 ist eine Stahlschmelze 3 enthalten, auf der eine Schlackeschicht 4 aufliegt. Zur Stahlherstellung wird durch den Boden des Konverters 1 durch Bodendüsen 5 Argon in die Metallschmelze eingeblasen. Von oben wird mittels einer Blaslanze 6 Sauerstoff eingeblasen. Neben der Blaslanze 6 wird eine sogenannte Sub-Lanze 7 in den Konverter eingeführt, an deren Eintauchende eine Messsonde 8 mit einem Messkopf 9 angeordnet ist.

Der Mess- bzw. Probennahmevorgang erfolgt während des Einblasens von Sauerstoff, in der Regel etwa 2 Minuten vor dem Ende des Sauerstoffeinblasens. Dabei wird die Temperatur gemessen und eine Probe zur Bestimmung, beispielsweise des Kohlenstoffgehaltes der Stahlschmelze, genommen. Anhand der Ergebnisse der Messungen kann eine Korrektur des Blasmodels erfolgen, um die Qualität der Stahlschmelze verändern zu können.

Nach dem Sauerstoff-Einblasen kann eine zweite Messung erfolgen. Dabei wird in der Regel neben der Temperatur der aktive Sauerstoffgehalt in der Stahlschmelze gemessen und es wird eine im Labor auszuwertende Probe zur Bestimmung der endgültigen Zusammensetzung des Stahls genommen. Anhand des Sauerstoffgehaltes kann innerhalb von wenigen Sekunden der aktuelle Kohlenstoffgehalt im Stahl bestimmt werden. Des Weiteren kann eine Vorkalkulation der benötigten Menge eines Desoxidationsmittels (Aluminium) erfolgen.

Neben dem bereits beschriebenen Effekt des Sauerstoffeinblasens und der damit verbundenen Gasblasen, die die Probennahme behindern oder stören, wirkt sich auch das durch die Bodendüsen 5 eingeblasene Gas unter Umständen störend auf die Probennahme aus, da dieses Gas aufsteigt in Richtung der eintauchenden Messsonde, so dass es genau auf die Messsonde und die Einlauföffnung trifft, wenn diese nicht geschützt ist. Des Weiteren gelangt durch die Aktivität der Blaslanze auch in geringem Umfang Schlacke in die Stahlschmelze hinein. Die Schlacke kann ebenfalls störend auf die Probennahme wirken.

Die erfindungsgemäße Ausbildung der Messsonde schafft hier Abhilfe, da Gasblasen und Schlacketeilchen durch den Schutzschild von der Einlauföffnung abgelenkt werden.

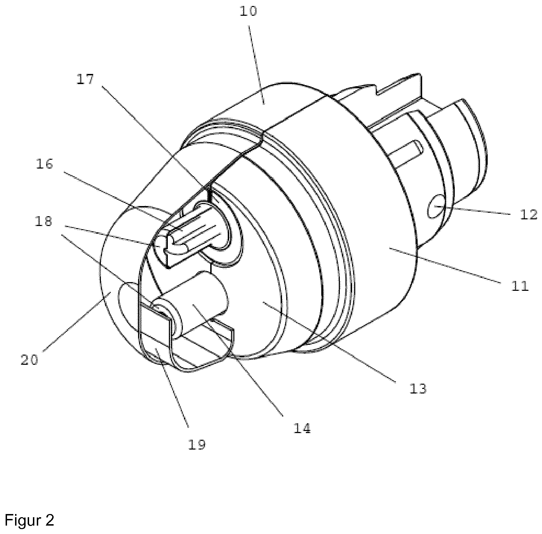

In Figur 2 ist der Messkopf dargestellt. Der Grundkörper des Messkopfes besteht aus zwei Teilen 10; 11 aus Gießereisand, die an ihrem dem Eintauchende abgewandten Ende eine Bohrung 12 aufweisen, durch die eine Schraube die beiden Teile 10; 11 aneinander fixiert. Diese zweiteilige Ausbildung erleichtert die Montage von Probenkammer und Sensoren.

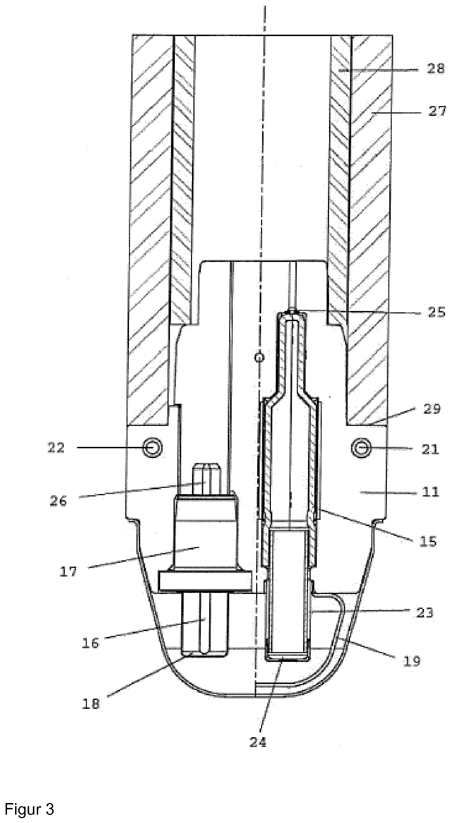

An der Stirnseite 13 des Messkopfes ist ein Einlaufkanal 14 vom inneren des Messkopfes nach außen geführt. Der Einlaufkanal ist im inneren des Messkopfes mit einer Probenkammer 15 (siehe Figur 3) verbunden. Ebenfalls an der Stirnseite 13 ist ein Thermoelement 16 angeordnet. Das Thermoelement 16 ist in einem Thermoelementeinsatz 17 fixiert. Sowohl das Thermoelement 16 als auch der Einlaufkanal 14 sind mit Schutzkappen 18 abgedeckt.

Neben dem Einlaufkanal 14 für die Probenkammer 15 ist ein Schutzschild 19 angeordnet. Der Schutzschild 19 ist aus einem abgebogenen Blechstreifen gebildet, der an der Stirnseite 13 des Messkopfes fixiert ist und der sich längs des Einlaufkanals erstreckt und vor der Einlauföffnung des Einlaufkanals abgebogen ist, so dass er die Einlauföffnung des Einlaufkanals 14 überdeckt. Der Abstand zwischen der Einlauföffnung und dem Schutzschild 19 ist etwas mehr als halb so groß wie der Durchmesser der Einlauföffnung. Die Einlauföffnung selbst ist in Figur 2 nicht dargestellt, da sie von einer Schutzkappe 18 abgedeckt ist.

An der Stirnseite 13 des Messkopfes ist eine Schlackekappe 20 angeordnet, die die Stirnseite 13 vollständig umfasst und abdeckt, einschließlich der an der Stirnseite angeordneten Elemente, das heißt des Thermoelementes 16 und des Einlaufkanals 14 mit den jeweiligen Schutzkappen 18 sowie einschließlich des Schutzschildes 19. Der Schutzschild 19 ist dadurch zwischen der Schutzkappe 18 des Einlaufkanals 14 und der Schlackekappe 20 angeordnet.

Das Innere des Messkopfes ist Figur 3 im Schnitt bzw. in der Ansicht auf die Schnittstelle eines Teils 11 des Messkopfes dargestellt. Beide Teile 10; 11 des Messkopfes werden aneinander durch Noppen 21, die in Vertiefungen 22 des jeweils anderen Teils eingreifen, aneinander justiert. Die in der Figur 3 dargestellte Probenkammer 15 ist eine Flachprobenkammer, die, wie weit verbreitet, mit zwei unterschiedlich dicken Teilen ausgebildet ist.

Der Einlaufkanal wird durch ein Quarzrohr 23 gebildet, dessen Einlauföffnung 24 von einer Schutzkappe 18 geschlossen ist. Das dem Einlaufkanal abgewandte Ende der Probenkammer 15 weist eine Entgasungsöffnung 25 auf, durch die das in der Probenkammer 15 befindliche Gas beim Einlaufen der Metallschmelze ausdringt. In der Figur 3 nicht dargestellt sind die an dem Kontaktstück 26 des Thermoelementeinsatzes 17 angeordneten Signalleitungen, die durch das rückseitige Ende des Messkopfes herausgeführt werden.

Der Messkopf ist auf Papprohre 27; 28 aufgesteckt und an dem äußeren Papprohr mittels eines feuerfesten Klebers 29 fixiert. Die Kombination der beiden Papprohre 27; 28 dient der Stabilisierung der Fixierung des Messkopfes.

## Patentansprüche

1. Messsonde zur Probennahme in Metallschmelzen, insbesondere in Stahlschmelzen, mit einem an einem Eintauchende eines Trägerrohres (27) angeordneten Messkopf (9), wobei der Messkopf (9) mindestens eine Probenkammer (15) aufweist, wobei die Probenkammer (15) einen Einlaufkanal (14) aufweist, dessen eines Ende in der Probenkammer (15) mündet und dessen zweites Ende eine Einlauföffnung (24) aufweist, die aus der dem Trägerrohr (27) abgewandten Stirnseite des Messkopfes (9) herausragt und mit einer Schutzkappe (18) abgedeckt ist, **dadurch gekennzeichnet, dass** außerhalb des Einlaufkanals (14) in Einlaufrichtung vor der Einlauföffnung (24) ein Schutzschild (19) beabstandet von der Einlauföffnung (24) angeordnet ist, wobei der Schutzschild (19) die Einlauföffnung (24) abdeckt und wobei der Schutzschild (19) den Einlaufkanal (14) seitlich nicht vollständig umgibt, und wobei die Stirnseite des Messkopfes (9) mit dem Einlaufkanal (14) und dem Schutzschild (19) von einer Schlackekappe (20) abgedeckt ist.

2. Messsonde nach Anspruch 1, **dadurch gekennzeichnet, dass** der Schutzschild (19) beständiger gegenüber Stahlschmelze ist als die Schutzkappe (18) der Einlauföffnung (24).

3. Messsonde nach Anspruch 1 oder 2, **dadurch gekennzeichnet, dass** der Schutzschild (19) an der Stirnseite des Messkopfes (9) angeordnet ist.

4. Messsonde nach einem der Ansprüche 1 bis 3, **dadurch gekennzeichnet, dass** der Schutzschild (19) als abgewinkeltes, streifenförmiges Material ausgebildet ist.

5. Messsonde nach einem der Ansprüche 1 bis 4, **dadurch gekennzeichnet, dass** der Schutzschild (19) aus Metall, Keramik oder Quarz gebildet ist.

6. Messsonde nach einem der vorhergehenden Ansprüche, **dadurch gekennzeichnet, dass** der Abstand zwischen Einlauföffnung (24) und Schutzschild (19) in Richtung des Einlaufkanals (14) mindestens halb so groß ist wie der Durchmesser der Einlauföffnung (24).

7. Messsonde nach einem der vorhergehenden Ansprüche, **dadurch gekennzeichnet, dass** an dem Messkopf (9) mindestens ein Sensor (16) zur Messung der Temperatur oder eines anderen Parameters der Metallschmelze angeordnet ist.

## Claims

1. A measuring probe for taking samples in molten metal, more particularly in molten steel, comprising a measuring head (9) arranged at an immersion end of a carrier tube (27), wherein the measuring head (9) has at least one sample cavity (15), wherein the sample cavity (15) has an inflow conduit (14) one end of which ends in the sample cavity (15) and the second end of which has an inflow opening (24) which projects from the front side of the measuring head (9) and is covered with a protective cap (18), said front side facing away from the carrier tube (27), **characterised in that** a protective shield (19) that is spaced apart from the inflow opening (24) is arranged outside the inflow conduit (14) and upstream of the inflow opening (24) in the inflow direction, wherein the protective shield (19) covers the inflow opening (24) and wherein the protective shield (19) does not entirely surround the inflow conduit (14) laterally, and wherein the front side of the measuring head (9) with the inflow conduit (14) and the protective shield (19) is covered with a slag cap (20).

2. The measuring probe according to claim 1, **characterised in that** the protective shield (19) is more resistant to molten steel than the protective cap (18) of the inflow opening (24).

3. The measuring probe according to claim 1 or 2, **characterised in that** the protective shield (19) is arranged on the front side of the measuring head (9).

4. The measuring probe according to any one of claims 1 to 3, **characterised in that** the protective shield (19) is formed as an angled strip-shaped material.

5. The measuring probe according to any one of claims 1 to 4, **characterised in that** the protective shield (19) is formed from metal, ceramic or silica.

6. The measuring probe according to any one of the preceding claims, **characterised in that** the distance between the inflow opening (24) and the protective shield (19) in the direction of the inflow conduit (14) is at least half the size of the diameter of the inflow opening (24).

7. The measuring probe according to any one of the preceding claims, **characterised in that** at least one sensor (16) for measuring the temperature or a different parameter of the molten metal is arranged at the measuring head (9).

## Revendications

1. Sonde de mesure pour le prélèvement d'échantillons dans des masses fondues métalliques, notamment dans des masses fondues d'acier, avec une tête de mesure (9) disposée à une extrémité d'immersion d'un tuyau de support (27), dans laquelle la tête de mesure (9) présente au moins une chambre d'échantillons (15), dans laquelle la chambre d'échantillons (15) présente un canal d'entrée (14) dont une extrémité débouche dans la chambre d'échantillons (15) et dont la seconde extrémité présente une ouverture d'entrée (24) qui dépasse du côté frontal de la tête de mesure (9) détourné du tuyau de support (27) et est recouverte d'un capuchon protecteur (18), **caractérisée en ce qu'**un panneau protecteur (19) est disposé à l'écart de l'ouverture d'entrée (24) en dehors du canal d'entrée (14) dans le sens d'entrée avant l'ouverture d'entrée (24), dans laquelle le panneau protecteur (19) recouvre l'ouverture d'entrée (24) et dans laquelle le panneau protecteur (19) n'entoure pas complètement latéralement le canal d'entrée (14), et dans laquelle le côté frontal de la tête de mesure (9) est recouvert avec le canal d'entrée (14) et le panneau protecteur (19) d'un capuchon à scorie (20).

2. Sonde de mesure selon la revendication 1, **caractérisée en ce que** le panneau protecteur (19) est plus résistant par rapport à la masse fondue d'acier que le capuchon protecteur (18) de l'ouverture d'entrée (24).

3. Sonde de mesure selon la revendication 1 ou 2, **caractérisée en ce que** le panneau protecteur (19) est disposé sur le côté frontal de la tête de mesure (9).

4. Sonde de mesure selon une des revendications 1 à 3, **caractérisée en ce que** le panneau protecteur (19) est réalisé en tant que matériau coudé en forme de bande.

5. Sonde de mesure selon une des revendications 1 à 4, **caractérisée en ce que** le panneau protecteur (19) est formé de métal, céramique ou quartz.

6. Sonde de mesure selon une des revendications précédentes, **caractérisée en ce que** l'écart entre l'ouverture d'entrée (24) et le panneau protecteur (19) en direction du canal d'entrée (14) est au moins la moitié du diamètre de l'ouverture d'entrée (24).

7. Sonde de mesure selon une des revendications précédentes, **caractérisée en ce qu'**au moins un capteur (16) pour la mesure de la température ou d'un autre paramètre de la masse fondue métallique est disposé sur la tête de mesure (9).
